(19) Europäisches Patentamt
European Patent Office
Office européen des brevets

(11) Publication number: **0 354 611 B1**

(12) **EUROPEAN PATENT SPECIFICATION**

(45) Date of publication of patent specification: **21.04.93**

(51) Int. Cl.[5]: **A61K 39/015**, G01N 33/569, //C07K7/10

(21) Application number: **89201984.5**

(22) Date of filing: **28.07.89**

The file contains technical information submitted after the application was filed and not included in this specification

(54) **Synthtic antigens useful in the diagnosis of malaria induced by plasmodium vivax.**

(30) Priority: **05.08.88 IT 2166488**

(43) Date of publication of application:
**14.02.90 Bulletin 90/07**

(45) Publication of the grant of the patent:
**21.04.93 Bulletin 93/16**

(84) Designated Contracting States:
**AT BE CH DE ES FR GB GR LI LU NL SE**

(56) References cited:
**EP-A- 0 223 711**
**WO-A-87/00533**

**THE JOURNAL OF BIOLOGICAL CHEMISTRY, vol. 262, no. 14, 15 May 1987, Baltimore, MD (US); V.F.DE LA CRUZ et al., pp. 6464-6467&NUM;**

**EXPERIENTIA, vol. 40, no. 12, December 1984, Birkhäuser Verlag, Basel (CH); L.PERRIN et al., pp. 1343-1350&NUM;**

**JOURNAL OF IMMUNOLOGY, vol. 146, no. 5, 1991; pp. 1674-1678&NUM;**

**SCIENCE, vol. 230, 15 November 1985; pp. 815-818&NUM;**

(73) Proprietor: **ENIRICERCHE S.p.A.**
**Corso Venezia 16**
**I-20121 Milan(IT)**

(72) Inventor: **Pessi, Antonello**
**Via Massaciuccoli 19**
**I-00199 Rome(IT)**
Inventor: **Verdini, Antonio Silvio**
**Via S. Martino 12**
**I-00015 Monterotondo Rome(IT)**

(74) Representative: **Roggero, Sergio et al**
**Ing. Barzanò & Zanardo Milano S.p.A. Via Borgonuovo 10**
**I-20121 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

This invention relates to new synthetic antigens useful in the malaria field.

More particularly, the invention relates to diagnostically useful synthetic peptides immunoreactive towards the antisporozoite antibodies of Plasmodium vivax and to immunological methods using them for the determination of malaria induced by Plasmodium vivax. The etiological agent of malaria is a protozoon of the Plasmodium genus which develops partly in the host vertebrate and partly in the vector mosquito in accordance with a multi-stage cycle.

The infection originates in man with the immission of Plasmodium sporozoites by the anopheles mosquito. Of the four species infective to man, Plasmodium falciparum and Plasmodium vivax are the most widespread and cause most of the morbidity and morality associated with said infection.

Malaria is currently in great upsurge, particularly in the tropical zones of Asia, Africa and America, because of the appearance and spread of drug-resistant parasites and the deterioration in control systems with consequent increase in elimination programmes.

In particular, Plasmodium vivax (P.vivax), which causes the so-called "benign tertian malaria", is widespread throughout the world and represents the dominant species in certain areas such as China, Sri Lanka and Vietnam.

Field screening of populations living in areas where malaria is endemic or in areas where careful control must be exercised to prevent or localize infective foci is therefore necessary to combat this disease.

An essential element in conducting an antimalaria campaign is to obtain information on the incidence of the infection before, during and at the end of such a campaign.

This consequently requires the availability of a diagnostic method suitable for large-scale studies while at the same time having a high level of specificity and sensitivity.

Methods are known in the art for malaria diagnosis by microscopic analysis of blood samples or by immunological methods using natural antigenic proteins.

These known methods however are hardly suitable for use in epidemological studies in which hundreds of thousands of samples are to be analyzed both because of the lengthy time required and because of the use of antigens which have little stability and are available only in limited quantity.

Recent developments in the molecular biology field have made it possible to obtain nucleotide and amino acid sequence characterization of antigens associated with various stages of development of the parasite.

In particular, the coding genes for the circumsporoitic protein (CSP) antigen of the sporozoitic stage of P.falciparum and of P.vivax have been cloned and sequentiated as reported in EP 166410 and WO 87/0533 respectively. This has enabled diagnostic methods to be set up based essentially on the use of peptides with an amino acid sequence corresponding to that of an immunodominant epitope contained in said antigens, and obtained by chemical synthesis or by recombinant DNA techniques as described, for example, by Del Guidice G. et al [J. Clin. Microbiol. 25, 91 (1987)], by H.K. Webster el al. [J. Clin. Microbiol. 25, 1002 (1987)] and in Italian patent application No. 23038 A/87.

This latter, in particular, describes and claims an ELISA method for determining anti-CSP antibodies of P.vivax based on the use of synthetic peptides of sequence $(Asp\text{-}Arg\text{-}Ala\text{-}Asp\text{-}Gly\text{-}Gln\text{-}Pro\text{-}Ala\text{-}Gly)_n$ where n is greater than 2, this corresponding to the nonapeptide repeating sequence present in the immunodominant region of the P.vivax CSP.

According to said patent application, the preferred peptide is that in which n is 4 ($V_4$), as it shows the best compromise between effectiveness of the assay and synthesis difficulty.

Said known peptides are however not completely satisfactory, particularly in view of their low selectivity towards the P.vivax anti-CSP antibodies.

In this respect, the P.vivax CSP amino acid sequence, deduced from the nucleotide sequence, shows a central repeating domain of 171 amino acids composed of a monomer of nine amino acids repeated 19 times in tandem.

In the same domain, two variants of said monomer are observed which differ from each other by the presence of the alanine residue (variation a) or of the aspartic acid residue (variant d) in position four.

In order to check whether the variations in said repeating monomers generate epitope differences, Romero et al. [J. Immunol. 139, 1679 (1987)] have conducted studies using a series of synthetic peptides with an amino acid sequence corresponding both to that of the a and d monomers and to that of two consecutive aa, dd and da monomers as competitive inhibitors in an RIA in which the specific binder was the recombinant protein rCSP containing part of the P.vivax CSP sequence including the entire repeated zone.

The results have shown that the antibody response towards the P.vivax CSP is heterogeneous, ie the monoclonal antibodies recognise not only the epitope entirely contained in the base nonapeptide but also epitopes containing a sequence involving two consecutive monomers.

Consequently, the synthetic peptides described in Italian patent application No. 23038 A/87, consisting of the repetition of only the d nonapeptide, are unable to detect those antibodies which selectively recognise the other epitopes contained in the P.vivax CS protein.

Peptides have now been synthesized with antigen characteristics similar to those of the immunodominant domain of the native CS protein of P.vivax which enable the problems of the known art to be obviated.

One object of the present invention is therefore to provide synthetic peptides immunoreactive towards Plasmodium vivax anti-CSP antibodies which can be used in diagnosing malaria induced by Plasmodium vivax.

A further object of the present invention is to use said synthetic peptides for evolving immunological diagnostic methods for detecting antisporozoite antibodies of Plasmodium vivax in a human blood sample.

Further objects of the invention will be apparent on reading the text and examples given hereinafter.

Specifically, the peptides according to the invention consist of five nonapeptides with an amino acid sequence corresponding to that of variant a or variant d of the repeating monomer of the circumsporozoitic protein of P.vivax in which each of said nonapeptides forms with that preceding it or with that following it all four combinations aa, dd, da and ad where:

a is Asp-Arg-Ala-Ala-Gly-Gln-Pro-Ala-Gly or in single-letter code DRAAGQPAG, and d is Asp-Arg-Ala-Asp-Gly-Gln-Pro-Ala-Gly or in single-letter code DRADGQPAG.

More specifically, the synthetic peptides according to the invention, indicated hereinafter as V5, are characterised by the following sequences:

- $(DRADGQPAG)_2$ - $(DRAAGQPAG)_2$ - DRADGQPAG, (ddaad)
- $(DRAAGQPAG)_2$ - $(DRADGQPAG)_2$ - DRAAGQPAG, (aadda)
- DRADGQPAG - $(DRAAGQPAG)_2$ - $(DRADGQPAG)_2$, (daadd)
- DRAAGQPAG - $(DRADGQPAG)_2$ - $(DRAAGQPAG)_2$, (addaa).

Peptides suitable for the purposes of the present invention can consist of a larger number of repetitions of the nonapeptides corresponding to the variants a and d.

Synthetic peptides according to the present invention can be prepared by using one of the known general techniques of peptide synthesis.

Preferably, said peptides are synthesized in the solid phase by the method described by A. Drylan and R.C. Sheppard [J. Chem. Soc. Perkin Trans. I, 125, (1986)] using a commerical functionalised polymerized polyacrylamide resin and an automatic synthesizer. This resin is packed into a glass column and the reagents added by a pump. This variant of the method known as the "Fmoc polyamide method" is called the "Flow polyamide method".

The synthesis strategy comprises adding one amino acid at a time using 4-hydroxymethylphenoxyacetic acid as the resin-peptide linking handle.

The proline and alanine residues in position 7 and 8 of the C-terminal nonapeptide are added in the form of the Pro-Ala dipeptide to prevent the base-catalyzed formation of the Ala-Gly diketopiperazine in position 8-9.

The Pro-Ala dipeptide is prepared in the homogeneous phase by known general methods.

According to the present invention the aminoacids are added to the resin after protecting the reactive functional groups.

Preferably the N-terminal amino group is protected by the fluorenylmethyloxycarbonyl (Fmoc) group, the aspartic acid $\beta$-carboxylicgroup by the tert-butyl ester ($OBu^t$) and the arginine guanidine group by the 4-methoxy-2,3,6-trimethylbenzylsulphonic (Mtr) group.

According to the present invention the first two reaction cycles, ie the esterification of the first amino acid at the handle and the subsequent condensation of the Pro-Ala dipeptide are conducted in a glass container (beaker) by bringing the suitably swollen resin into contact with the necessary reactants in N,N-dimethylformamide (DMF).

For esterification, the amino acid protected at the N-terminal is hooked to the resin in the presence of a catalyst such as 4-dimethylaminopyridine (DMAP) at ambient temperature (20-25 °C). The resin is then washed with DMF, methanol and ethyl ether and dried.

The dipeptide is then condensed onto the resin swollen with DMF and treated with a solution of piperidine in DMF, operating in the presence of 1-hydroxybenzotriazole (HOBt) and dicyclohexylcarbodiimide (DCCI) at ambient temperature (20-25 °C).

The resin thus treated is then packed into a column and the peptides are then synthesized according to the present invention using an automatic synthesizer.

Each synthesis cycle consists of a stage in which the protector group is released from the N-terminal group of the amino acid and a stage in which the next amino acid residue is acylated.

In the release stage the resin is washed with DMF and then treated with the piperidine/DMF (20% v/v) solution.

In the acylation stage the active amino acid (pentafluorophenol ester for glutamine, symmetrical anhydride for all other amino acids) is dissolved in DMF and then added to the resin.

In all cases the completion of the condensation of the amino acids onto the resin is monitored by the colorimetric test described by Kaiser E et al. [Analyt. Biochem., 34, 595 (1980)] and by Hancock W.S. and Battersby J.E. [Analyt. Biochem., 71, 260 (1976)].

After the synthesis the aminoterminal protector group is released from the peptide, the resin is removed from the column and the peptide released from the resin by treatment with trifluoroacetic acid (TFA) to which phenol has been added (5% w/v), at ambient temperature.

The resin is removed from the obtained suspension by filtration or centrifuging, washed repeatedly with TFA and water, again filtered and the pooled filtrates evaporated under vacuum to remove the TFA.

The aqueous solution containing the peptide is then extracted with ethyl ether and the organic phase re-extracted with water.

The pooled aqueous phases are finally lyophilized and then purified by gel filtration chromatography operating by known methods.

Typically a chromatograph column packed with Sephadex® G-25 resin is used, eluting with 0.1 M acetic acid at a throughput of 36 ml/hour.

The fractions corresponding to the required peptide are collected and lyophilized.

According to the present invention said peptides have been used in a classical ELISA for detecting anti-CSP antibodies of P.vivax in human blood samples originating from endemic malaria zones.

The results obtained, given in Example 2 below, show that said peptides are able to detect anti-CSP antibodies of P.vivax in samples which give a negative result with the known peptide V4. Consequently peptides according to the present invention are particularly useful in evolving immunological methods of RIA, IFA or EIA type which are of high sensitivity and specificity in detecting anti-CSP antibodies of P.vivax in human blood samples. Diagnostic kits for the immunological determination of P.vivax antisporozoite antibodies in a human blood sample can also be developed using the synthetic peptides of the present invention, both as specific binders on an insoluble support and as competitive inhibitors.

A technical expert of the art would have no difficulty in establishing the most suitable operating conditions and reactants for developing a method and diagnostic kit according to the present invention.

The experimental examples given hereinafter illustrate but do not limit the invention.

## EXAMPLE 1

### Synthesis of the V5 peptide ddaad

The peptide was synthesized in the solid phase by the method described by Drylan A. and Sheppard R.C. [J. Chem. Soc. Perkin trans. I 125 (1986)] using the automatic synthesizer BIOLYNX® 4170 (Pharmacia-LKB) and 2.5 g of polyacrylamide resin polymerized within the pores of a rigid macroporous matrix of Kieselguhr, Ultrosyn KA, with a nominal functionalization of 0.1 milli-equivalents/g.

The amino acids were introduced one at a time after suitably protecting the N-terminal amino group by means of the fluorenylmethyloxycarbonyl (Fmoc) group, the aspartic acid $\beta$-carboxyl group by the tert-butyl ester ($OBu^t$) and the arginine guanidine group by the 4-methoxy-2,3,6-trimethylbenzyl-sulphonic (Mtr) group.

The first two reaction cycles, ie the esterification of the first amino acid at the handle and the subsequent condensation of the dipeptide $Pro^{43}$-$Ala^{44}$ were conducted by suspending the resin, previously swollen in N,N-dimethylformamide (DMF), and the reactants dissolved in a total volume of 5 ml of DMF, in a 20 ml capacity beaker.

For esterification, 1.3 mmoles (0.380 mg) of $(Fmoc\text{-}Gly)_2O$ and 0.13 mmoles of 4-dimethylaminopyridine (DMAP) as catalyst were used. The reaction was conducted at 25 °C for 30 minutes without agitation.

The resin was then washed with DMF, methanol and ethyl ether and dried under vacuum.

One aliquot was subjected to amino acid analysis and it was found that the functionalisation, after the connection of the first amino acid, was 0.062 mmoles/g of resin (85% incorporation).

For the dipeptide, the resin was swollen in DMF, treated twice with 15 ml of a solution of piperidine in DMF (20% v/v) for 10 minutes, and washed several times with DMF.

After removing the excess solvent, the acylating solution containing the Fmoc-Pro-Ala-OH dipeptide (1.3 mmoles, 0.525 g), 1-hydroxybenzotriazole (HOBt, 1.56 mmoles, 0.21 g) and dicyclohexylcarbodiimide (DCCI, 1.3 mmoles, 0.267 g) (a total of 5 ml DMF) was added. The resin was left for 16 hours at 25°C without stirring.

After this time the reaction was checked for completion by the ninhydrin test [E. Kaiser et al., Analyt. Biochem., 34, 595 (1980)] and the trinitrobenzenesulphonic acid test [W.S. Hancock and J.E. Battersby, Analyt. Biochem., 71, 260 (1976)].

The resin was then packed into a column of dimensions 11 x 1 cm and the remaining amino acids were synthesized by the BIOLYNX® automatic synthesizer using a cycle comprising a release stage for the protector group of the N-terminal amino acid and an acylation stage for the next residue.

In practice, for the release stage the resin was washed with DMF by pumping the solvent through the column for 5 minutes at a throughput of 4 ml/minute, then treated with piperidine/DMF (20% v/v) for 10 minutes at a throughput of 4 ml/minute, and finally washed with DMF for 10 minutes at a throughput of 4 ml/minute.

For the acylation stage, the amino acid in active form (pentafluorophenol ester for the glutamide and symmetrical anhydride for the other amino acids) was dissolved in 5 ml of DMF at a concentration of 0.36 mmoles/ml and then added to the resin.

The system was then put under recirculation for 45 minutes, testing for reaction completion at the end of the period by the aforesaid tests (yields > 99.5%).

In the case of the amino acid residues $Ala^{26}$ and $Gly^{14}$ the acylation was repeated, a positive result in the colorimetric tests being obtained after the second acylation.

The protector group for the terminal amino group was released after introducing the last amino acid.

The resin was then unpacked from the column, washed with DMF, methanol and ethyl ether and dried under nitrogen.

The final resin weight was 2.63 g.

The peptide was then released from the resin by treatment, per gram of resin, with 50 ml of trifluoroacetic acid (TFA) containing 5% (weight/volume) of phenol at 25°C for 6 hours.

The resin was then removed from the reaction mixture by filtration, washed with TFA and then with water, and the pooled filtrates evaporated under vacuum to remove the TFA.

The aqueous solution containing the peptide (100 ml) was extracted with ethyl ether (70 ml) and the organic phase re-extracted with water (70 ml). The pooled aqueous phases were lyophilized.

The release percentage was 97.2%.

The entire lyophilate resulting from the resin release was dissolved in 5 ml of 0.1 M $Ch_3COOH$, filtered and made up to a final volume of 22 ml with $CH_3COOH$.

The resultant solution was fed into a column (85 x 2.6 cm) packed with Sephadex® G-25 resin and eluted with 0.1 M $CH_3COOH$ at a throughput of 36 ml/hour.

The fractions corresponding to the required peptide were collected and lyophilized to obtain 348 mg (0.81 mmoles, 54%) of peptide.

Table I gives the results of the analysis for amino acid content during various stages of the synthesis and purification.

The V5 peptides daadd and aadda were synthesized by the aforesaid procedure.

TABLE I

| SAMPLE | Asp | Pro | Gln | Ala | Gly | Arg | NLc |
|---|---|---|---|---|---|---|---|
| $Asp^{38}$ | 1.89 (2) | 0.71 (1) | 1.00 | 2.04 (2) | 2.03 (2) | 1.12 (1) | 1.26 |
| $Asp^{28}$ | 2.80 (3) | 1.64 (2) | 2.00 | 5.08 (5) | 4.21 (4) | 2.29 (2) | 1.51 |
| $Asp^{19}$ | 3.93 (4) | 3.13 (3) | 3.00 | 7.51 (8) | 5.85 (6) | 3.26 (3) | 1.32 |
| $Asp^{10}$ | 5.88 (6) | 3.77 (4) | 4.00 | 10.10 (10) | 7.95 (8) | 4.12 (4) | 1.61 |
| Resin -peptide | 7.42 (8) | 5.07 (5) | 5.00 | 11.64 (12) | 9.61 (10) | 4.76 (5) | 1.51 |
| V5 after G-25 | 7.51 (8) | 5.06 (5) | 5.00 | 11.79 (12) | 10.11 (10) | 4.81 (5) | --- |
| N.B. The theoretical values are given in parentheses | | | | | | | |

EXAMPLE 2

ELISA for detecting antisporozoite antibodies of Plasmodium vivax in human serum samples

The V5 peptides, suspended in distilled water and stored at -70°C in microaliquots, were diluted in pH 7.8 PBS (phosphate buffered saline) to a final concentration of 3ug/ml. 100 µl of each solution were introduced into each microwell of a flat-bottomed 96-well plate of Nunc IMMUNOPLATE® 1 model (Nunc, Roskilde, Denmark).

The plates were then incubated in a humid chamber at ambient temperature (20-25°C) overnight.

The plates were then washed 4 times for 2-3 minutes each time with 100 µl of PBS containing 0.05% (v/v) of Tween® 20 (PBS-T).

The residual binding sites of the plate were then saturated by introducing into each well 200 µl of PBS-T containing 5% (w/v) of delipidized powdered milk as inert protein (PBS-T-M 5%).

After removing the unreacted inert protein by washing with PBS, 100 µl of serum samples originating from endemic malaria zones diluted 1:100 in PBS-T-M 2.5% were seeded into each well.

The plates treated in this matter were kept at ambient temperature for 1 hour and then washed (4 times for 2-3 minutes) with 100 µl of PBS-T.

100 µl of a solution of PBS-T containing the antibody/anti-human-immunoglobulin/peroxidase (Bio Rad) conjugate diluted 1:3000 were then introduced into each well.

The plates were kept at ambient temperature for 1 hour and then washed with PBS-T (4 times for 2-3 minutes).

Each well was then fed with 100 µl of pH 5.0 0.1 M citrate buffer containing 0.01% $H_2O_2$ and the enzymatic substrate orthophenylenediamine at a concentration of 0.4 mg/ml.

The enzymatic reaction was conducted at ambient temperature, and blocked after 30 minutes by adding 2.5 N sulphuric acid.

The optical density (OD) of the individual wells was determined by absorbance at 492 nm using a micro-ELISA reader.

Each experiment also used,in parallel, (uncoated) plates without the adsorbed V5 peptide, which were incubated with PBS alone and then processed as described for the (coated) plates pretreated with peptides.

The results, expressed as the difference (ΔOD) between the optical densities measured for one and the same sample in the coated plates and in the uncoated plates, are given in Table 1.

The high specificity of the V5 peptides is apparent from a comparison with the results obtained using the V4 synthetic antigen.

TABLE 1

| | | PEPTIDE | | | |
|---|---|---|---|---|---|
| | | dddd | daadd | aadda | ddaad |
| COUNTRY | SERUM | V4 | V5 | V5 | V5 |
| Vietnam | 1 | .870 | .741 | .607 | .715 |
| | 2 | .158 | .161 | .178 | .163 |
| | 3 | .316 | .683 | .780 | .867 |
| | 4 | .167 | .184 | .182 | .227 |
| | 5 | .107 | .068 | .109 | .104 |
| | 6 | .101 | .459 | .753 | .780 |
| | 7 | .260 | .303 | .434 | .557 |
| | 8 | .173 | .034 | .084 | .063 |
| | 9 | .028 | .268 | .297 | .300 |
| | 10 | .191 | .305 | .402 | .398 |
| | 11 | .122 | .084 | .199 | .065 |
| | 12 | .062 | 1.284 | 1.641 | 1.660 |
| | 13 | .094 | .047 | .264 | .127 |
| | 14 | .179 | .338 | .371 | .461 |
| | 15 | .178 | .165 | .461 | .406 |
| | 16 | .207 | .441 | .549 | .486 |
| | 17 | .410 | 1.427 | 1.278 | 1.131 |
| | 18 | .033 | .099 | .060 | .066 |
| | 19 | .521 | .409 | .450 | .387 |
| | 20 | .056 | .243 | .277 | .233 |

| | | | | | |
|---|---|---|---|---|---|
| China | 19 | .039 | .044 | .031 | .037 |
| | 30 | .045 | .187 | .102 | .050 |
| | 57 | .106 | .373 | .471 | .560 |
| | 37 | .033 | .055 | .086 | .065 |
| | 24 | .091 | .059 | .044 | .039 |
| | 60 | .074 | .031 | .079 | .052 |
| | 21 | .127 | .068 | .048 | .029 |
| | 8 | .035 | .261 | .434 | .429 |
| | 3 | .117 | .114 | .105 | .116 |
| | 18 | .050 | .279 | .667 | .613 |
| | 40 | .174 | .023 | .095 | .087 |
| | 54 | .078 | .000 | .053 | .030 |
| | 61 | .147 | .172 | .176 | .152 |
| Sri Lanka | M/90/3 | .536 | .880 | 1.136 | .969 |
| | M/90/10 | .058 | .776 | .992 | 1.092 |
| | M/90/7 | .018 | .101 | .147 | .199 |
| | M/90/2 | .000 | .138 | .217 | .216 |
| | M/182/2 | .135 | .276 | .226 | .236 |
| | M/193/1 | .186 | .286 | .261 | .263 |
| | M/198/2 | .389 | .650 | .526 | .585 |
| | K1/25-12 | 1.242 | 1.259 | 1.135 | .970 |
| | M/243/3 | .166 | .318 | .314 | .222 |
| | M/144/1 | .012 | .008 | .045 | .024 |
| | M/192/1 | .054 | .075 | .070 | .122 |
| | M/134/2 | .249 | .412 | .720 | .445 |
| | G/53/1 | .202 | .417 | .504 | .400 |

| | | | | |
|---|---|---|---|---|
| G/53/5 | .244 | .353 | .405 | .337 |
| G/53/9 | .079 | .000 | .000 | .000 |
| Cut-off values (1) | .300 | .250 | .360 | .330 |

(1) Cut-off value = average of O.D. values + 3 standard deviations determined on a sample of 100 sera from healthy blood donors, collected in Geneva.

## Claims

### Claims for the following Contracting States : AT, BE, CH, DE, FR, GB, LI, LU, NL, SE

1. A five-nonapeptide synthetic peptide immunoreactive towards antisporozoite antibodies of Plasmodium vivax for detecting said antibodies in human blood samples, containing both the aminoacid sequences:
(DRAAGQPAG) = variant a, and
(DRADGQPAG) = variant d,
of the repeating monomer of the circumsporozoitic protein of Plasmodium vivax, each of said nonapeptides forming, with its preceding nonapeptide and with its following nonapeptide, all the combinations:
aa, dd, ad, and da.

2. A synthetic peptide according to Claim 1, wherein the aminoacid sequence of which it consists is:

$$(DRADGQPAG)_2-(DRAAGQPAG)_2-(DRADGQPAG)$$

3. A synthetic peptide according to Claim 1, wherein the aminoacid sequence of which it consists is:

$$(DRAAGQPAG)_2-(DRADGQPAG)_2-(DRAAGQPAG)$$

4. A synthetic peptide according to Claim 1, wherein the aminoacid sequence of which it consists is:

$$(DRADGQPAG)-(DRAAGQPAG)_2-(DRADGQPAG)_2$$

5. A synthetic peptide according to Claim 1, wherein the aminoacid sequence of which it consists is:

$$(DRAAGQPAG)-(DRADGQPAG)_2-(DRAAGQPAG)_2$$

6. Use of a synthetic peptide as defined in Claims 1 to 5 for detecting, by an immunological method selected from Immunoenzymatic Assay (EIA), Immunoradiometric Assay (RIA) and Immunofluorometric Assay (IFA), antisporozoite antibodies of Plasmodium vivax in a human blood sample.

7. Use of a synthetic peptide according to Claim 6, wherein the Immunoenzymatic Assay method is ELISA.

8. Use of a synthetic peptide as defined in Claim 1 to 5, in a diagnostic kit for the immunological detection of antisporoite antibodies of Plasmodium vivax in a human blood sample.

**Claims for the following Contracting States : ES, GR**

1. Process for preparing a five-nonapeptide synthetic peptide immunoreactive towards antisporozoite antibodies of Plasmodium vivax for detecting said antibodies in human blood samples, containing both the aminoacid sequences:
(DRAAGQPAG) = variant a, and
(DRADGQPAG) = variant d,
of the repeating monomer of the circumsporozoitic protein of Plasmodium vivax, each of said nonapeptides forming, with its preceding nonapeptide and with its following nonapeptide, all the combinations:
aa, dd, ad, and da.
characterized in that it comprises the steps of synthesizing the expected synthetic peptide in the solid phase in an automatic synthesizer, in the presence of a polyacrylamide resin embedded in a siliceous matrix, by introducing the aminoacids forming the expected sequence one at a time, after having chemically protected, wherever present, the N-terminal amino group, the aspartic acid betacarbonyl group, and the arginine guanidine group, and separating the as formed peptide from the resin.

2. Process according to Claim 1, characterized in that the aminoacid sequence is:

$$(DRADGQPAG)_2—(DRAAGQPAG)_2—(DRADGQPAG)$$

3. Process according to Claim 1, characterized in that the aminoacid sequence is:

$$(DRAAGQPAG)_2—(DRADGQPAG)_2—(DRAAGQPAG)$$

4. Process according to Claim 1, characterized in that the aminoacid sequence is:

$$(DRADGQPAG)—(DRAAGQPAG)_2—(DRADGQPAG)_2$$

5. Process according to Claim 1, characterized in that the aminoacid sequence is:

$$(DRAAGQPAG)—(DRADGQPAG)_2—(DRAAGQPAG)_2$$

**Patentansprüche**
**Patentansprüche für folgende Vertragsstaaten : AT, BE, CH, DE, FR, GB, LI, LU, NL, SE**

1. Synthetisches Fünf-Nonapeptid, das gegenüber Antisporozoit-Antikörpern von Plasmodium vivax zum Auffinden dieser Antikörper in Humanblutproben immunreaktiv ist, welches sowohl die Aminosäuresequenz:
(DRAAGQPAG) = Variante a
als auch
(DRADGQPAG) = Variante d
des wiederkehrenden Monomers des Circumsporozoit-Proteins von Plasmodium vivax, enthält, wobei jedes dieser Nonapeptide mit seinem vorangehenden Nonapeptid und mit seinem nachfolgenden Nonapeptid alle Kombinationen
aa, dd, ad und da

ausbildet.

2. Synthetisches Peptid nach Anspruch 1, worin die es ausbildende Aminosäuresequenz

$$(DRADGQPAG)_2—(DRAAGQPAG)_2—(DRADGQPAG)$$

ist.

3. Synthetisches Peptid nach Anspruch 1, worin die es ausbildende Aminosäuresequenz

$$(DRAAGQPAG)_2—(DRADGQPAG)_2—(DRAAGQPAG)$$

ist.

4. Syntehtisches Peptid nach Anspruch 1, worin die es ausbildende Aminosäuresequenz

$$(DRADGQPAG)—(DRAAGQPAG)_2—(DRADGQPAG)_2$$

ist.

5. Synthetisches Peptid nach Anspruch 1, worin die es ausbildende Aminosäuresequenz

$$(DRAAGQPAG)—(DRADGQPAG)_2—(DRAAGQPAG)_2$$

ist.

6. Verwendung eines synthetischen Peptids, wie in den Ansprüchen 1 bis 5 definiert, zum Feststellen von Antisporozoit-Antikörpern von Plasmodium vivax in einer Humanblutprobe nach einer immunologischen Methode, die unter Enzymimmunoassay (EIA), Radioimmunoassay (RIA) und Fluoroimmunoassay (FIA) ausgewählt ist.

7. Verwendung eines synthetischen Peptids nach Anspruch 6, worin die immunoenzymatische Untersuchungsmethode ELISA ist.

8. Verwendung eines synthetischen Peptids nach Anspruch 1 bis 5 in einem diagnostischen Kit zur immunologischen Detektion von Antisporozoit-Antikörpern von Plasmodium vivax in einer Humanblutprobe.

**Patentansprüche für folgende Vertragsstaaten : ES, GR**

1. Verfahren zur Herstellung eines gegenüber Antisporozoit-Antikörpern von Plasmodium vivax immunreaktiven synthetischen Fünf-Nonapeptids zur Feststellung dieser Antikörper in Humanblutproben, welches Peptid sowohl die Aminosäuresequenz:
(DRAAGQPAG) = Variante a
als auch
(DRADGQPAG) = Variante d
des wiederkehrenden Monomers des Circumsporozoit-Proteins von Plasmodium vivax enthält, wobei jedes dieser Nonapeptide mit seinem vorangehenden Nonapeptid und mit seinem nachfolgenden

Nonapeptid alle Kombinationen

aa, dd, ad und da

ausbildet, dadurch gekennzeichnet, daß es die Stufen des Synthetisierens des erwarteten synthetischen Peptids in fester Phase in einem automatischen Synthesizer in Anwesenheit eines in einer Kieselsäurematrix eingebetteten Polyacrylamidharzes durch Einführen der die gewünschte Sequenz ausbildenden Aminosäuren, einer nach der anderen, nach einem chemischen Schützen der N-endständigen Aminogruppe, der Asparaginsäure-Betacarbonylgruppe und der Arginin-Guanidingruppe, soferne vorhanden, und ein Abtrennen des gebildeten Peptids von dem Harz umfaßt.

2. Verfahren nach Anspruch 1, dadurch gekennzeichnet, daß die Aminosäuresequenz

$$(DRADGQPAG)_2—(DRAAGQPAG)_2—(DRADGQPAG)$$

ist.

3. Verfahren nach Anspruch 1, dadurch gekennzeichnet, daß die Aminosäuresequenz

$$(DRAAGQPAG)_2—(DRADGQPAG)_2—(DRAAGQPAG)$$

ist.

4. Verfahren nach Anspruch 1, dadurch gekennzeichnet, daß die Aminosäuresequenz.

$$(DRADGQPAG)—(DRAAGQPAG)_2—(DRADGQPAG)_2$$

ist.

5. Verfahren nach Anspruch 1, dadurch gekennzeichnet, daß die Aminosäuresequenz

$$(DRAAGQPAG)—(DRADGQPAG)_2—(DRAAGQPAG)_2 ,$$

ist.

**Revendications**

**Revendications pour les Etats contractants suivants : AT, BE, CH, DE, FR, GB, LI, LU, NL, SE**

1. Peptide synthétique à cinq nonapeptides, immunoréactif vis-à-vis d'anticorps anti-sporozoïtes de Plasmodium vivax, destiné à la détection de ces anticorps dans des échantillons de sang humain, contenant les deux séquences d'acides aminés :

   (DRAAGQPAG) = variante a, et

   (DRADGQPAG) = variante d,

   du monomère répété de la protéine circumsporozoïtique de Plasmodium vivax, chacun de ces nonapeptides formant, avec le nonapeptide qui le précède et avec celui qui le suit, toutes les combinaisons :

   aa, dd, ad, et da.

2. Peptide synthétique conforme à la revendication 1, constitué par la séquence d'acides aminés suivante :

   $(DRADGQPAG)_2$-$(DRAAGQPAG)_2$-(DRADGQPAG)

3. Peptide synthétique conforme à la revendication 1, constitué par la séquence d'acides aminés suivante :
(DRAAGQPAG)$_2$-(DRADGQPAG)$_2$-(DRAAGQPAG)

4. Peptide synthétique conforme à la revendication 1, constitué par la séquence d'acides aminés suivante :
(DRADGQPAG)-(DRAAGQPAG)$_2$-(DRADGQPAG)$_2$

5. Peptide synthétique conforme à la revendication 1, constitué par la séquence d'acides aminés suivante :
(DRAAGQPAG)-(DRADGQPAG)$_2$-(DRAAGQPAG)$_2$

6. Utilisation d'un peptide synthétique tel que défini dans l'une des revendications 1 à 5 pour la détection, par une méthode immunologique choisie parmi une méthode immunoenzymatique (EIA), une méthode immunoradiométrique (RIA) et une méthode immunofluorimétrique (IFA), d'anticorps anti-sporozoïtes de Plasmodium vivax dans un échantillon de sang humain.

7. Utilisation d'un peptide synthétique selon la revendication 6, dans laquelle la méthode immunoenzymatique est la méthode ELISA.

8. Utilisation d'un peptide synthétique tel que défini dans l'une des revendications 1 à 5 dans une trousse de diagnostic pour la détection immunologique d'anticorps anti-sporozoïtes de Plasmodium vivax dans un échantillon de sang humain.

**Revendications pour les Etats contractants suivants : ES, GR**

1. Procédé de préparation d'un peptide synthétique à cinq nonapeptides, immunoréactif vis-à-vis d'anticorps anti-sporozoïtes de Plasmodium vivax, destiné à la détection de ces anticorps dans des échantillons de sang humain, contenant les deux séquences d'acides aminés :
(DRAAGQPAG) = variante a, et
(DRADGQPAG) = variante d,
du monomère répété de la protéine circumsporozoïtique de Plasmodium vivax, chacun de ces nonapeptides formant, avec le nonapeptide qui le précède et avec celui qui le suit, toutes les combinaisons :
aa, dd, ad, et da,
caractérisé en ce qu'il comprend les étapes consistant à synthétiser le peptide synthétique voulu, sur phase solide, dans un appareil de synthèse automatique, en présence d'une résine de polyacrylamide enrobée dans une matrice siliceuse, en introduisant les acides aminés formant la séquence voulue, un seul à la fois, après avoir chimiquement protégé, chaque fois que l'un de ces groupes est présent, le groupe amino N-terminal, le groupe $\beta$-carbonyle de l'acide aspartique et le groupe guanidino de l'arginine, et à séparer le peptide formé de la résine.

2. Procédé conforme à la revendication 1, caractérisé en ce que la séquence d'acides aminés est la suivante :
(DRADGQPAG)$_2$-(DRAAGQPAG)$_2$-(DRADGQPAG)

3. Procédé conforme à la revendication 1, caractérisé en ce que la séquence d'acides aminés est la suivante :
(DRAAGQPAG)$_2$-(DRADGQPAG)$_2$-(DRAAGQPAG)

4. Procédé conforme à la revendication 1, caractérisé en ce que la séquence d'acides aminés est la suivante :
(DRADGQPAG)-(DRAAGQPAG)$_2$-(DRADGQPAG)$_2$

5. Procédé conforme à la revendication 1, caractérisé en ce que la séquence d'acides aminés est la suivante :
(DRAAGQPAG)-(DRADGQPAG)$_2$-(DRAAGQPAG)$_2$